# EUROPEAN PATENT APPLICATION

(11) **EP 4 851 131 A1**
(43) Date of publication of application: **09.12.2026**
(21) Application number: 25748483.2
(22) Date of filing: 15.01.2025
(51) Int. Cl.: C07C 63/24, C07C 63/26, C07C 211/05, C07D 207/34, C07F 5/00, C07F 5/06

(54) **METHOD FOR PRODUCING METAL ORGANIC FRAMEWORK**

(30) Priority: 31.01.2024 JP 2024012717; 24.10.2024 JP 2024187585
(71) Applicant: SUMITOMO CHEMICAL COMPANY, LIMITED, Tokyo 103-6020 (JP)
(72) Inventor: OKADA, Takeshi, Ichihara-shi, Chiba 299-0195 (JP); KIKUCHI, Yuta, Ichihara-shi, Chiba 299-0195 (JP)
(74) Representative: Vossius & Partner Patentanwälte Rechtsanwälte mbB
(86) International application number: PCT/JP2025/000986
(87) International publication number: WO 2025/164303

(57) **Abstract**

An object of the present disclosure is to provide a process for obtaining a metal organic framework by reacting an organic compound with a metal salt in a water-based solvent, the process being capable of producing the metal organic framework having good filterability. The present disclosure relates to a process for producing a metal organic framework, comprising:
continuously or intermittently adding, to a reaction vessel, one or more selected from A and B, A being a polyvalent carboxylic acid or an alkali metal salt of a polyvalent carboxylic acid, and B being a metal compound; and
mixing A, which is the polyvalent carboxylic acid or the alkali metal salt of the polyvalent carboxylic acid, and the metal compound B, in the reaction vessel in a solvent D containing water in the presence of a base C, wherein
a pH of a mixture in the reaction vessel is maintained at 6 or less until 0.3 equivalents of the metal compound B come into contact with 0.3 equivalents of A, which is the polyvalent carboxylic acid or the alkali metal salt of the polyvalent carboxylic acid, in the reaction vessel, and is also maintained at 6 or less until 0.8 equivalents of the metal compound B come into contact with 0.8 equivalents of A, which is the polyvalent carboxylic acid or the alkali metal salt of the polyvalent carboxylic acid.

## Description

### TECHNICAL FIELD

The present disclosure relates to a process for producing a metal organic framework.

### BACKGROUND ART

Metal organic frameworks are also referred to as porous coordination polymers and are a class of materials which form porous structures by coordinate bonds between metal ions and organic ligands, and are expected to adsorb and desorb gas and to be applied to catalysts, etc.

Metal organic frameworks can be produced by reacting a polyvalent carboxylic acid serving as an organic ligand with a metal compound in a solvent, followed by heat treatment for a predetermined period of time as required.

For example, Patent Literature 1 discloses that a metal organic framework was obtained by mixing biphenyl-3,3', 5,5'-tetracarboxylic acid, Al(NO₃)₃·9H₂O, and piperazine, dispersing the mixture in water, adding nitric acid to the resulting white slurry, transferring the slurry to an autoclave, sealing the autoclave, and heating the slurry at 210°C for three days.

### CITATION LIST

### PATENT LITERATURE

Patent literature 1 : International Publication No. WO2013/144628

### SUMMARY OF THE INVENTION

### DISCLOSURE OF THE INVENTION

Processes for obtaining a metal organic framework by reacting a polyvalent carboxylic acid with a metal compound in a solvent include a method in which an organic solvent such as N,N-dimethylformamide is used as a solvent. However, as disclosed in Patent Literature 1, a process for producing a metal organic framework in which water is used as the solvent is advantageous in terms of improving productivity, since a step of removing the organic solvent is not required.

However, the present inventors have found through studies that, in the process for obtaining a metal organic framework by reacting a polyvalent carboxylic acid with a metal compound in a water-based solvent as disclosed in Patent Literature 1, the filterability may be poor when the metal organic framework is taken out from the reaction liquid by filtration.

Accordingly, an object of the present disclosure is to provide a process for obtaining a metal organic framework by reacting a polyvalent carboxylic acid with a metal compound in a water-based solvent, the process being capable of producing the metal organic framework having good filterability.

### SOLUTION TO THE PROBLEMS

The present disclosure for achieving the above object is as follows.
[1] A process for producing a metal organic framework, comprising:
   continuously or intermittently adding, to a reaction vessel, one or more selected from A and B, A being a polyvalent carboxylic acid or an alkali metal salt of a polyvalent carboxylic acid, and B being a metal compound; and
   mixing A, which is the polyvalent carboxylic acid or the alkali metal salt of the polyvalent carboxylic acid, and the metal compound B, in the reaction vessel in a solvent D containing water in the presence of a base C, wherein
   a pH of a mixture in the reaction vessel is adjusted to 6 or less by the time 0.3 equivalents of the metal compound B come into contact with 0.3 equivalents of A, which is the polyvalent carboxylic acid or the alkali metal salt of the polyvalent carboxylic acid, in the reaction vessel, and the pH of the mixture in the reaction vessel is maintained at 6 or less until 0.8 equivalents of the metal compound B come into contact with 0.8 equivalents of A, which is the polyvalent carboxylic acid or the alkali metal salt of the polyvalent carboxylic acid.
[2] The process according to [1], wherein
   a difference between a maximum pH value and a minimum pH value during a period from when 0.3 equivalents of the metal compound B come into contact with 0.3 equivalents of A, which is the polyvalent carboxylic acid or the alkali metal salt of the polyvalent carboxylic acid, to when 0.8 equivalents of the metal compound B come into contact with 0.8 equivalents of A, which is the polyvalent carboxylic acid or the alkali metal salt of the polyvalent carboxylic acid, is 4 or less.
[3] The process according to [1] or [2], wherein an acid-base mixture obtained by mixing A, which is the polyvalent carboxylic acid or the alkali metal salt of the polyvalent carboxylic acid, with at least a portion of the base C is added continuously or intermittently to the reaction vessel.
[4] The process according to any one of [1] to [3], wherein
   an acid-base mixture containing A, which is the polyvalent carboxylic acid or the alkali metal salt of the polyvalent carboxylic acid, and a portion of the base C, is added continuously or intermittently to the reaction vessel via a first addition port, and a portion of the base C is added continuously or intermittently to the reaction vessel via a second addition port while addition of the acid-base mixture is continued.
[5] The process according to [3] or [4], wherein the metal compound B is previously charged into the reaction vessel, and the acid-base mixture is added to the reaction vessel.
[6] The process according to [3] or [4], wherein the metal compound B is added continuously or intermittently to the reaction vessel via a third addition port while the addition of the acid-base mixture is continued.
[7] The process according to any one of [1] to [3], wherein
   an acid-base mixture containing A, which is the polyvalent carboxylic acid or the alkali metal salt of the polyvalent carboxylic acid, and at least a portion of the base C, is added continuously or intermittently to the reaction vessel via a first addition port, and the metal compound B is added continuously or intermittently to the reaction vessel via a third addition port while addition of the acid-base mixture is continued.
[8] The process according to any one of [1] to [7], wherein a content of water in the solvent D is 70 mass% or more, based on 100 mass% of the solvent D.

### ADVANTAGEOUS EFFECTS OF THE INVENTION

According to the production process of the present disclosure, it is possible to obtain a metal organic framework having good filterability.

### BRIEF DESCRIPTION OF THE DRAWINGS

[FIG. 1] FIG. 1 is a graph showing pH behavior in Example 1-1 described below.
[FIG. 2] FIG. 2 is a graph showing pH behavior in Comparative Example 1 described below.

### DESCRIPTION OF EMBODIMENTS

The present disclosure is directed to a process for producing a metal organic framework, and the process includes continuously or intermittently adding, to a reaction vessel, one or more selected from A and B, A being a polyvalent carboxylic acid or an alkali metal salt of a polyvalent carboxylic acid (hereinafter, sometimes referred to as "a polyvalent carboxylic acid or a salt thereof A") and B being a metal compound, and mixing A, which is the polyvalent carboxylic acid or the salt thereof, and the metal compound B, in the reaction vessel in a solvent D containing water in the presence of a base C. First, A, which is the polyvalent carboxylic acid or the salt thereof, the metal compound B, the base C, and the solvent D containing water, each of which is used in the present disclosure, will be described.

### [Polyvalent carboxylic acid or alkali metal salt of polyvalent carboxylic acid, as A]

A polyvalent carboxylic acid is a compound represented by R(COOH)ₙ (in which R is an n-valent group and n is an integer of 2 or more), and one type thereof or two or more types thereof may be used. R may be an aliphatic acyclic hydrocarbon group, an alicyclic hydrocarbon group, an aliphatic heterocyclic hydrocarbon group (a group in which one or more carbon atoms of an alicyclic hydrocarbon group are replaced with heteroatoms), an aromatic hydrocarbon group, or an aromatic heterocyclic hydrocarbon group (a group in which one or more carbon atoms of an aromatic hydrocarbon group are replaced with heteroatoms). An aromatic hydrocarbon group and/or an aromatic heterocyclic hydrocarbon group are preferred, and an aromatic hydrocarbon group is more preferred. The number of carbon atoms in R may be 2 to 30, may be 4 to 24, and is preferably 6 to 18. n may be 2 or more and 4 or less, and is preferably 2 or more and 3 or less, and more preferably 2. It is preferable that R is an aromatic hydrocarbon group and/or an aromatic heterocyclic hydrocarbon group and has 4 to 24 carbon atoms.

The above-described aliphatic acyclic hydrocarbon groups, alicyclic hydrocarbon groups, aliphatic heterocyclic hydrocarbon groups, aromatic hydrocarbon groups, and aromatic heterocyclic hydrocarbon groups may further contain one or more functional groups X, which are -OH and/or -NH₂.

The aliphatic acyclic hydrocarbon group may be straight-chain or branched, and may be a saturated hydrocarbon group or an unsaturated hydrocarbon group. Examples of the aliphatic acyclic hydrocarbon group include groups obtained by removing n hydrogen atoms from ethane, ethylene, acetylene, butane, butene, or hexane.

Examples of the alicyclic hydrocarbon group include groups obtained by removing n hydrogen atoms from cyclopropane, cyclobutane, cyclohexane, cyclooctane, norbornene, or adamantane.

Examples of the aliphatic heterocyclic hydrocarbon group include groups obtained by removing n hydrogen atoms from pyrrolidine, piperidine, piperazine, or morpholine.

The preferred ranges of the number of carbon atoms in the aromatic hydrocarbon group are, in order, 6 or more and 30 or less, 6 or more and 24 or less, 6 or more and 18 or less, 6 or more and 12 or less, and 6 or more and 10 or less. Specifically, a group obtained by removing n hydrogen atoms (preferably two or three hydrogen atoms) from benzene or biphenyl is preferable, and a group obtained by removing n hydrogen atoms (preferably two or three hydrogen atoms) from benzene is particularly preferable.

The aromatic hydrocarbon group may be represented by any one of the following formulas (A-1) to (A-9), preferably any one of the following formulas (A-1) to (A-3) and (A-5), more preferably the following formula (A-1), (A-2), or (A-5), and still more preferably the following formula (A-1) or (A-2). In the following formulas (A-1) to (A-9), * represents a bonding site, and the group is bonded to -COOH at *.

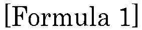

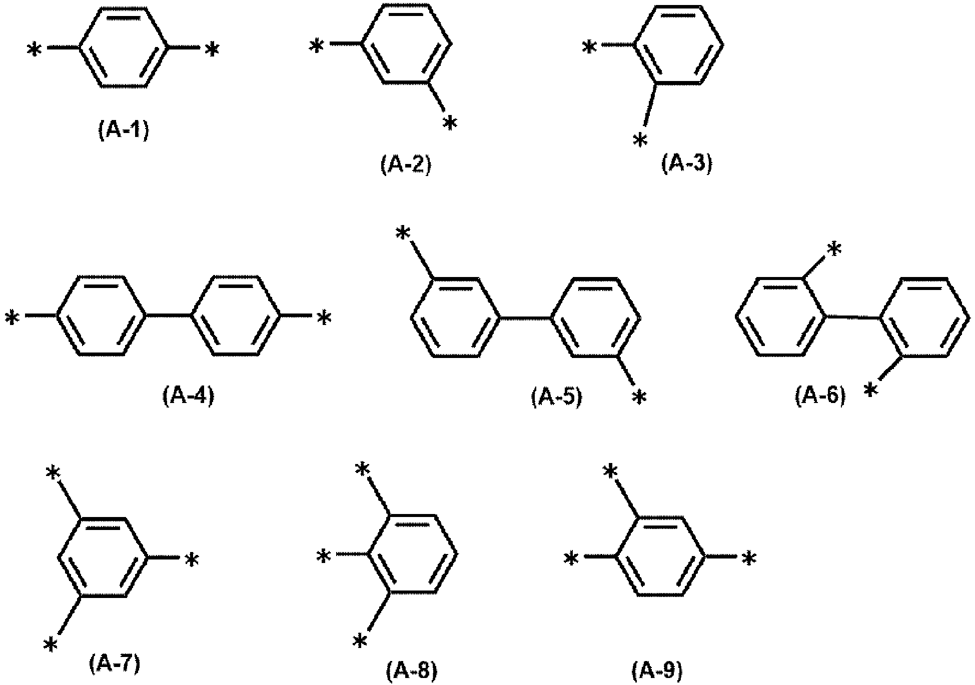

In the above formulas (A-1) to (A-9), at least one hydrogen atom bonded to a carbon atom may be substituted with -OH and/or -NH₂, or hydrogen atoms bonded to carbon atoms may remain unsubstituted.

When the polyvalent carboxylic acid A contains one or two of R(COOH)₂ in which R is represented by formula (A-1) or (A-2), the total content of R(COOH)₂ in which R is represented by formula (A-1) or (A-2), based on 100 mass% of the polyvalent carboxylic acid A, is preferably 80 mass% or more, more preferably 90 mass% or more, and most preferably 100 mass% (i.e., preferably 80 to 100 mass%, and more preferably 90 to 100 mass%).

Examples of the aromatic heterocyclic hydrocarbon group include groups obtained by removing n hydrogen atoms (e.g., two or three hydrogen atoms) from pyrrole, pyrazole, imidazole, thiazole, oxazole, pyridine, pyrimidine, pyridazine, pyrazine, or triazine, and preferred examples include groups obtained by removing n hydrogen atoms (e.g., two or three hydrogen atoms) from pyrrole or pyrazine.

The polyvalent carboxylic acid preferably contains one or more types of R(COOH)ₙ in which R is an aromatic hydrocarbon group, and the total amount of one or more types of R(COOH)ₙ in which R is an aromatic hydrocarbon group, based on 100 mass% of the polyvalent carboxylic acid, may be more than 50% on a molar basis, is preferably 80% or more, and more preferably 100% (i.e., preferably more than 50% and 100% or less, and more preferably 80 to 100%). The polyvalent carboxylic acid also preferably contains one or more types of R(COOH)ₙ in which R is an aromatic hydrocarbon group and one or more types of R(COOH)ₙ in which R is an aromatic heterocyclic hydrocarbon group, and particularly preferably one or two of R(COOH)₂ in which R is represented by the above formula (A-1) or (A-2) and one or more types of R(COOH)ₙ in which R is a group obtained by removing n hydrogen atoms from pyrrole or pyrazine (particularly, n is 2). When the polyvalent carboxylic acid contains one or more types of R(COOH)ₙ in which R is an aromatic hydrocarbon group and one or more types of R(COOH)ₙ in which R is an aromatic heterocyclic hydrocarbon group, the total amount of one or more types of R(COOH)ₙ in which R is an aromatic hydrocarbon group and one or more types of R(COOH)ₙ in which R is an aromatic heterocyclic hydrocarbon group relative to the total amount of the polyvalent carboxylic acid may be 80% or more on a molar basis, and is preferably 100%. When the polyvalent carboxylic acid contains one or more types of R(COOH)ₙ in which R is an aromatic hydrocarbon group and one or more types of R(COOH)ₙ in which R is an aromatic heterocyclic hydrocarbon group, the total amount of one or more types of R(COOH)ₙ in which R is an aromatic hydrocarbon group relative to the total amount of one or more types of R(COOH)ₙ in which R is an aromatic hydrocarbon group and one or more types of R(COOH)ₙ in which R is an aromatic heterocyclic hydrocarbon group may be more than 50% on a molar basis, may be 60% or more, is preferably 70% or more, and may be 90% or less (i.e., preferably more than 50% and 90% or less, more preferably 60 to 90%, and still more preferably 70 to 90%).

The alkali metal salt of the polyvalent carboxylic acid is preferably an Li salt, a Na salt, a K salt, or a Rb salt of the above-described polyvalent carboxylic acid, more preferably a Na salt, and still more preferably a Na salt of R(COOH)ₙ in which R is an aromatic heterocyclic hydrocarbon group, and further preferably a Na salt of a group obtained by removing n hydrogen atoms (particularly, 2 hydrogen atoms) from pyrazine.

A, which is the polyvalent carboxylic acid or the salt thereof, is preferably a polyvalent carboxylic acid A (i.e., A does not contain alkali metal salts of the polyvalent carboxylic acid).

### [Metal compound B]

Metal in the metal compound B is, for example, at least one selected from the group consisting of elements of Groups 3 to 12 in Periods 4 to 6 of the periodic table, as well as Al, Ga, and In. The metal may be at least one selected from the group consisting of Al, Ga, In, Ti, Zr, Hf, V, Cr, Mn, Fe, Co, Ni, Cu, and Zn, and is preferably at least one selected from the group consisting of Al, In, Cu, and Zn, and more preferably Al. In addition, the metal compound B may be a sulfate, a nitrate, an acetate, a perchlorate, a chloride, a bromide, or an alkoxide of the metal, preferably a sulfate or a perchlorate, and more preferably a sulfate. Any of the metal compounds may be a hydrate. The metal compound particularly preferably contains a sulfate of Al, that is, Al₂(SO₄)₃, and Al₂(SO₄)₃ may be a hydrate. One type of the metal compound B may be used, or two or more types thereof may be used.

The molar amount of the metal in the metal compound B may be 0.8 to 1.2 mol per 1 mol of the polyvalent carboxylic acid represented by R(COOH)ₙ or a salt thereof.

With respect to A, which is the polyvalent carboxylic acid or salt thereof, and the metal compound B, the above-described preferred embodiments may be combined in any manner. However, combinations other than those in which the polyvalent carboxylic acid is 2,4-pyridinedicarboxylic acid and the metal compound is CoCl₂ are preferred.

### [Base C]

In the present disclosure, in order to obtain a metal organic framework by reacting A, which is the polyvalent carboxylic acid or the salt thereof, with the metal compound B in the solvent D containing water, it is important that the reaction is performed in the presence of the base C. When the reaction is performed without using the base C, the metal organic framework may not be obtained, or the amount of impurities other than the metal organic framework may increase.

The base C may be an inorganic base or an organic base. One type of the base C may be used, or two or more types thereof may be used.

Examples of the inorganic base include hydroxides, carbonates, hydrogen carbonates, phosphates, carboxylates, and alkoxides of alkali metals or alkaline earth metals. Alkali metal hydroxides are preferred, and NaOH is more preferred.

Examples of the organic base include triethylamine, N,N-diisopropylethylamine, piperidine, pyridine, 4-(N,N-dimethylamino)pyridine, and pyrazine. Triethylamine or pyrazine is preferred, and triethylamine is more preferred.

The amount of the base C (the total amount when two or more types are used) may be 1.5 to 5 mol or 2 to 4 mol, per 1 mol of A, which is the polyvalent carboxylic acid or the salt thereof (the total amount when two or more types are used).

### [Solvent D]

The solvent D contains water. The content of water may be 70 mass% or more and 100 mass% or less, 80 mass% or more and 100 mass% or less, or 100 mass%, based on 100 mass% of the solvent D. Examples of solvents other than water, which may be contained in the solvent D, include organic solvents that are one or more of alcohol-based solvents, amide-based solvents, ketone-based solvents, ester-based solvents, and ether-based solvents. Amide solvents are preferred. Examples of the amide solvents include N,N-dimethylformamide and N,N-dimethylacetamide.

Next, the adjustment of pH in the production process of the present disclosure and specific procedures in preferred embodiments will be described in detail. In the production process of the present disclosure, it is important that the pH of a mixture in a reaction vessel is adjusted to 6 or less by the time 0.3 equivalents of the metal compound B come into contact with 0.3 equivalents of A, which is the polyvalent carboxylic acid or the salt thereof, in the reaction vessel, and that the pH of the mixture in the reaction vessel is maintained at 6 or less until 0.8 equivalents of the metal compound B come into contact with 0.8 equivalents of A, which is the polyvalent carboxylic acid or the salt thereof, in the reaction vessel.

As used herein, 0.3 equivalents of each of the metal compound B and A, which is the polyvalent carboxylic acid or the salt thereof, mean 0.25 equivalents or more and less than 0.35 equivalents, 0.8 equivalents of each of the metal compound B and A, which is the polyvalent carboxylic acid or the salt thereof, mean 0.75 equivalents or more and less than 0.85 equivalents, and a pH of 6 or less means a pH of less than 6.5.

In the production process of the present disclosure, the pH of the mixture in the reaction vessel is adjusted to less than 6.5 by the time 0.35 equivalents of the metal compound B come into contact with 0.35 equivalents of A, which is the polyvalent carboxylic acid or the alkali metal salt of the polyvalent carboxylic acid, and the pH of the mixture in the reaction vessel remains at less than 6.5 until 0.85 equivalents of the metal compound B come into contact with 0.85 equivalents of A, which is the polyvalent carboxylic acid or the alkali metal salt of the polyvalent carboxylic acid.

One equivalent of the metal compound B and one equivalent of A, which is the polyvalent carboxylic acid or the salt thereof, respectively refer to the molar amount of the metal compound B and the molar amount of A, which is the polyvalent carboxylic acid or the salt thereof, that are capable of theoretically forming the maximum amount of the metal organic framework from the metal compound B and A, which is the polyvalent carboxylic acid or the salt thereof, used.

The pH of the mixture in the reaction vessel is adjusted to 6 or less by the time 0.3 equivalents of the metal compound B come into contact with 0.3 equivalents of A, which is the polyvalent carboxylic acid or the salt thereof(hereinafter, sometimes simply referred to as "0.3 equivalent contact"), and the pH of the mixture in the reaction vessel is maintained at 6 or less until 0.8 equivalents of the metal compound B come into contact with 0.8 equivalents of A, which is the polyvalent carboxylic acid or the salt thereof (hereinafter, sometimes simply referred to as "0.8 equivalent contact"). As a result, filterability of the resulting metal organic framework can be improved, which is presumably because the metal in the metal compound B can exist in the form of a metal ion (e.g., in the case of Al, Al³⁺) during the reaction of the polyvalent carboxylic acid or the salt thereof A with the metal compound B. As long as the above requirements are satisfied, the pH before the time of the 0.3 equivalent contact after the start of contact between the metal compound B and A, which is the polyvalent carboxylic acid or the salt thereof, and the pH after 0.8 equivalent contact, are not particularly limited.

The pH to be adjusted by the time of the 0.3 equivalent contact and the pH to be maintained until the 0.8 equivalent contact are each preferably 5.5 or less, and more preferably 5.0 or less, and the lower limit may be, for example, 2.5 or 3.0 (i.e., 5.5 to 2.5 is preferred, and 5.0 to 3.0 is more preferred). Setting the pH within such ranges is preferable in view of improving the filterability of the resulting metal organic framework.

In addition, the difference between the maximum pH value and the minimum pH value during the period from the 0.3 equivalent contact to the 0.8 equivalent contact (i.e., during the period from the 0.25 equivalent contact to the 0.85 equivalent contact) is preferably 4 or less, more preferably 3 or less, and still more preferably 2 or less. Although the smaller the difference is, the more preferable it is, the difference may be 0.01 or more (i.e., 4 to 0.01 is preferred, 3 to 0.01 is more preferred, and 2 to 0.01 is still more preferred). Setting the difference between the maximum pH value and the minimum pH value within such ranges is preferable in view of improving the filterability of the resulting metal organic framework.

Furthermore, the difference between the maximum pH value and the minimum pH value during the period from the 0.35 equivalent contact to the 0.85 equivalent contact is preferably 4 or less, more preferably 3 or less, and still more preferably 2 or less. Although the smaller the difference is, the more preferable it is, the difference may be 0.01 or more (i.e., 4 to 0.01 is preferred, 3 to 0.01 is more preferred, and 2 to 0.01 is still more preferred).

Continuously or intermittently adding one or more selected from A and a metal compound B to the reaction vessel, wherein A is the polyvalent carboxylic acid or the salt thereof, means that the total amount thereof is not added all at once but is added over a predetermined period of time. The average addition rate of A, which is the polyvalent carboxylic acid or the salt thereof, from the start to the end of addition is, for example, 1 mmol/min to 10 mmol/min. The average addition rate of the metal compound B from the start to the end of addition is, for example, 0.5 mmol/min to 5 mmol/min.

More specifically, in the production process of the present disclosure, an acid-base mixture obtained by mixing A, which is the polyvalent carboxylic acid or the salt thereof, with at least a portion of the base C is preferably added continuously or intermittently to the reaction vessel (embodiment (x)). The acid-base mixture may contain a salt formed from the polyvalent carboxylic acid and the base. According to embodiment (x), A, which is the polyvalent carboxylic acid or the salt thereof, and the base C can be added uniformly to the reaction vessel, and the reaction of A, which is the polyvalent carboxylic acid or the salt thereof, with the metal compound B can proceed uniformly in the solvent D, which is preferable.

In embodiment (x), A, which is the polyvalent carboxylic acid or the salt thereof, may be mixed with all of the base C (embodiment (x1)), or A, which is the polyvalent carboxylic acid or the salt thereof, may be mixed with a portion of the base C (embodiment (x2)).

In either of embodiments (x1) and (x2) (i.e., throughout embodiment (x)), while the addition of the acid-base mixture is continued (via a first addition port), the metal compound B may be added continuously or intermittently to the reaction vessel via another addition port (third addition port); or the metal compound B may be previously charged into the reaction vessel, and the acid-base mixture may be added to the reaction vessel. In embodiment (x1), it is also preferable that the metal compound B is previously charged into the reaction vessel and the acid-base mixture is added to the reaction vessel.

Among embodiments (x), embodiment (x2) is preferred. In embodiment (x2), while the addition of the acid-base mixture is continued, a portion of the base C (which refers to a base different from the base C that is mixed with A, which is the polyvalent carboxylic acid or the salt thereof, and which may correspond to the entire remaining amount of the base C after mixing with A, which is the polyvalent carboxylic acid or the salt thereof, or a portion thereof) may be added continuously or intermittently to the reaction vessel via another addition port (embodiment (x2-1));
or may be previously charged into the reaction vessel, and the acid-base mixture may be added into this reaction vessel (embodiment (x2-2)), and embodiment (x2-1) is more preferred. That is, in embodiment (x2-1), the acid-base mixture containing A, which is the polyvalent carboxylic acid or a salt thereof, and the portion of the base C is added continuously or intermittently to the reaction vessel via the first addition port, and the portion of the base C is added continuously or intermittently to the reaction vessel via a second addition port while the addition of the acid-base mixture is continued. In embodiment (x2), the portion of the base that is different from the base C mixed with A, which is the polyvalent carboxylic acid or the salt thereof, may be of the same type as, or of a different type from, the base C mixed with the polyvalent carboxylic acid or the salt thereof.

In any of embodiments (x1), (x2-1), and (x2-2), the metal compound B (a portion or the entirety thereof) may be previously charged into the reaction vessel; while the addition of the acid-base mixture is continued, the metal compound B (a portion or the entirety thereof) may be added continuously or intermittently to the reaction vessel via the other addition port (third addition port); or a portion of the metal compound B may be previously charged into the reaction vessel, and the remaining metal compound B other than the metal compound B previously charged into the reaction vessel may be added continuously or intermittently to the reaction vessel via the other addition port (third addition port) while the addition of the acid-base mixture is continued.

In particular, in embodiment (x2-1), while the addition of the acid-base mixture is continued, the metal compound B (in particular, the entirety of the metal compound B) is preferably added continuously or intermittently to the reaction vessel via the other addition port (third addition port).

When, while the addition of the acid-base mixture is continued, the base C and/or the metal compound B are added continuously or intermittently to the reaction vessel via the other addition port (second or third addition port), "while the addition of the acid-base mixture is continued" may refer to the entirety of the time period during which the addition of the acid-base mixture is continued, or a portion thereof.

In the present specification, the first to third addition ports are different from each other, which means that the addition ports are not identical.

In the production process of the present disclosure, a strong acid (e.g., sulfuric acid or nitric acid) or a buffer solution (e.g., a phosphate buffer solution, 2-amino-2-(hydroxymethyl)propane-1,3-diol, citric acid, phosphoric acid, diethylbarbituric acid, or boric acid), and the polyvalent carboxylic acid may be previously charged into the reaction vessel, and then the metal compound may be added to the reaction vessel (embodiment y). In this case, the base C may be previously charged into the reaction vessel together with the polyvalent carboxylic acid or the like, or may be added to the reaction vessel together with the metal compound. In addition, the solvent D containing water may be previously charged into the reaction vessel together with the polyvalent carboxylic acid or the like, or a mixture of the metal compound and the solvent D may be added to the reaction vessel. It is preferred that the solvent D containing water is previously charged into the reaction vessel together with the polyvalent carboxylic acid or the like, and a mixture of the metal compound and the solvent D is added to the reaction vessel.

In the present disclosure, including preferred embodiments, A, which is the polyvalent carboxylic acid or the salt thereof, the metal compound B, and the base C are each preferably mixed with the solvent D to form a liquid mixture and then added to the reaction vessel. In this case, the solvent D may or may not be previously charged into the reaction vessel.

In the present disclosure, including preferred embodiments, the amount of A, which is the polyvalent carboxylic acid or the salt thereof, the amount of the metal compound B, and the amount of the base C are preferably within the following ranges, respectively.

The amount of A, which is the polyvalent carboxylic acid or the salt thereof, per 1 L of the solvent D may be 0.05 to 1.5 mol, or 0.1 to 1 mol.

The amount of the metal compound B per 1 L of the solvent D may be 0.01 to 1 mol, or 0.05 to 0.7 mol.

The amount of the base C per 1 L of the solvent D may be 0.1 to 3 mol, or 0.5 to 2.5 mol.

In particular, all of the following are preferably satisfied: the amount of A, which is the polyvalent carboxylic acid or the salt thereof, is 0.1 to 1 mol; the amount of the metal compound B is 0.05 to 0.7 mol; and the amount of the base C is 0.5 to 2.5 mol; per 1 L of the solvent D.

In the production process of the present disclosure, additives other than A, which is the polyvalent carboxylic acid or the salt thereof, the metal compound B, the base C, and the solvent D may be included. The amount of the additives may be 3 parts by mass or less, 2 parts by mass or less, or 1 part by mass or less, based on 100 parts by mass of the total amount of A, which is the polyvalent carboxylic acid or the salt thereof, the metal compound B, the base C, and the solvent D, and the amount is preferably 0 parts by mass (i.e., 3 to 0 parts by mass is preferred, 2 to 0 parts by mass is more preferred, and 1 to 0 parts by mass is still more preferred).

In the present disclosure, the temperature at which A, which is the polyvalent carboxylic acid or the salt thereof, and the metal compound B are mixed may be, for example, 15 to 45°C, or 20 to 30°C. After the entire amount of A, which is the polyvalent carboxylic acid or the salt thereof, and the entire amount of the metal compound B are mixed (preferably, after all of A, which is the polyvalent carboxylic acid or the salt thereof, the metal compound B, the base C, and the solvent D are mixed), the resulting mixture is preferably heated, for example, at 50 to 150°C for 5 to 30 hours, and more preferably at 70 to 120°C for 8 to 20 hours. The pressure during the heating may be 2.0 MPa or less, 1.8 MPa or less, or 1.7 MPa or less, and may be atmospheric pressure (about 0.1 MPa) (i.e., 2.0 to 0.1 MPa is preferred, 1.8 to 0.1 MPa is more preferred, and 1.7 to 0.1 MPa is still more preferred). After the heating, a suspension is usually obtained, and the suspension is filtered, washed with water, and dried at 60 to 100°C for about 5 to 20 hours, whereby a metal organic framework can be obtained.

The specific filtration resistance of the metal organic framework obtained by the production process of the present disclosure, as evaluated according to the methods described in Examples below, can be reduced to 80% or less, preferably 70% or less, more preferably 60% or less, and particularly preferably 30% or less, as compared to a case where the same types and amounts of A, which is the polyvalent carboxylic acid or the salt thereof, the metal compound B, the base C, and the solvent D are used without performing the pH adjustment of the present disclosure. The specific value of the specific filtration resistance is, for example, 5.0×10¹² (m/kg) or less, preferably 1.0×10¹¹ (m/kg) or less, more preferably 7.0×10¹⁰ (m/kg) or less, and still more preferably 5.0×10¹⁰ (m/kg) or less, and may be 5.0×10⁹ (m/kg) or more (i.e., for example, 5.0×10¹² to 5.0×10⁹ (m/kg); 1.0×10¹¹ to 5.0×10⁹ (m/kg) is preferred; 7.0×10¹⁰ to 5.0×10⁹ (m/kg) is more preferred; and 5.0×10¹⁰ to 5.0×10⁹ (m/kg) is still more preferred.

The BET specific surface area of the metal organic framework obtained by the production process of the present disclosure is, for example, 130 to 850 m²/g, preferably 600 m²/g to 850 m²/g, more preferably 650 m²/g to 850 m²/g, and particularly preferably 700 m²/g to 850 m²/g. The BET specific surface area of the metal organic framework is also preferably 600 m²/g to 700 m²/g.

The metal organic framework produced by the production process of the present disclosure is suitably used, for example, for the adsorption and removal of gases and organic molecules. Examples of gases include water (water vapor), carbon dioxide, hydrogen, carbon monoxide, oxygen, nitrogen, hydrocarbons having 1 to 4 carbon atoms, noble gases, hydrogen sulfide, ammonia, sulfur oxides, nitrogen oxides, and siloxanes. Examples of organic molecules include hydrocarbons having 5 to 8 carbon atoms, alcohols having 1 to 8 carbon atoms, aldehydes having 1 to 8 carbon atoms, carboxylic acids having 1 to 8 carbon atoms, ketones having 1 to 8 carbon atoms, amines having 1 to 8 carbon atoms, esters having 1 to 8 carbon atoms, and amides having 1 to 8 carbon atoms. The organic molecules may contain aromatic rings.

This application claims priority to Japanese Patent Application No.2024-012717 filed on January 31, 2024, and Japanese Patent Application No. 2024-187585 filed on October 24, 2024. The entire contents of Japanese Patent Application No.2024-012717 filed on January 31, 2024, and Japanese Patent Application No. 2024-187585 filed on October 24, 2024, are incorporated herein by reference.

### EXAMPLES

Hereinafter, the present disclosure will be described more specifically by means of Examples. The present disclosure is not limited by the following Examples, and can also be carried out with appropriate modifications being made within the scope of the gist described above and below, and any of these modifications are included in the technical scope of the present disclosure.

The following Examples and Comparative Examples were evaluated by the methods described below.

### (1) Calculation of specific filtration resistance

A metal filter capable of being internally pressurized and filter paper (qualitative filter paper manufactured by Whatman, Grade 2, diameter of 47 mm) were used. The suspension after the reaction was subjected to pressure filtration under a pressure of 40 kPa at a measurement temperature of 25°C, and the filtration rate v [m/s] was measured. The specific filtration resistance αₘ [m/kg] was calculated from the results on the basis of Ruth's equation.

### (2) BET specific surface area

Since the adsorption cross-sectional area of the nitrogen molecules is known in advance, the amount of gas molecules adsorbed only on the surface of a sample was measured, and the surface area of the sample was measured based on the BET adsorption isotherm.

Sample preparation: To remove water contained in the sample, the sample was heated overnight under conditions of 200°C and a reduced pressure of 10 Pa or less.

Apparatus: BELSORP-mini manufactured by MicrotracBEL

### Pretreatment conditions:

(a-i) A volume-reducing glass rod (for a standard sample tube) was placed in a standard sample tube, and the tube was sealed with a quick seal. Sets of such sample tubes (up to 3 samples per measurement) were prepared for samples to be measured, and were connected to a pretreatment apparatus (BELPREP VACII), and, after the air inside the sample tubes was evacuated, N₂ gas (purity: 99.999% or higher) was introduced therein until the pressure inside the sample tubes reached atmospheric pressure;
(a-ii) Thereafter, each sample tube was removed from the pretreatment apparatus, and the weight was measured three times using a precision balance (that displays four or more decimal places) to obtain an average value (W1). When the precision balance was used, an ionizer was used to eliminate the effect of the static electricity;
(a-iii) About 50 mg of the sample to be measured was weighed on weighing paper and was directly placed into a spherical part in the lower part of the standard sample tube using a long stem funnel;
(a-iv) The glass rod was returned into the sample tube, the sample tube was sealed with a quick seal, and the total weight was measured once to preliminarily confirm the amount of the added sample;
(a-v) The sample tube containing the sample was connected to the pretreatment apparatus and the inside of the sample tube was evacuated; and
(a-vi) After the interior of the sample tube reached a sufficiently low pressure, heating was started (while evacuation was continued).

### Measurement conditions:

(b-i) After completion of the pretreatment (vacuum heating), the sample tube was allowed to cool while being maintained under reduced pressure. After the sample tube reached room temperature, N₂ gas was introduced therein until the pressure inside the sample tube reached atmospheric pressure, and the sample tube was removed from the apparatus;
(b-ii) The weight of the sample tube containing the sample after the pretreatment was measured three times using the precision balance to obtain an average value (W2). The weight of the sample was obtained by calculating W2-W1; and
(b-iii) The weight of the sample and information on N₂ gas at liquid nitrogen temperature (second virial coefficient, etc.) were inputted into the measurement software, a relative pressure for measurement was inputted, and a measurement start button was pressed. Thereafter, according to the software's instructions, a Dewar vessel filled with liquid nitrogen and the sample tube were installed, and the measurement was also performed.

### (3) Measurement of pH

The liquid in the reaction vessel was partially sampled at each of a plurality of time points during the dropwise addition of the solution, and the pH was measured using a pH meter (personal pH meter PH71-11JAA, manufactured by Yokogawa Electric Corporation).

### (4) Identification of metal organic framework

The substances produced in Examples and Comparative Examples were measured using a powder X-ray diffraction (PXRD) apparatus (manufactured by Rigaku Corporation) under the following conditions, and peak patterns derived from the metal organic framework were confirmed.
Radiation source: Cu
Measurement range: 2θ = 3 to 40°
Step size: 0.01°
Scanning rate: 3°/min
Measurement temperature: room temperature (25°C)

### Example 1-1

Isophthalic acid in an amount of 65.0 mmol, 143 mmol of triethylamine, and 238 mL of water were mixed to prepare a solution A. Al₂(SO₄)₃·nH₂O (n = 14 to 18) in an amount of 33.8 mmol and 31.5 mL of water were mixed to prepare a solution B. At 20 to 30°C, the dropwise addition of the solution A, the solution B, and 52 mmol of triethylamine into a reactor was simultaneously started, and each addition was carried out over 30 minutes. Thereafter, the mixture in the reactor was heated at 90°C for 12 hours to obtain a suspension. A portion of the obtained suspension was subjected to pressure filtration to determine the specific filtration resistance. The remaining suspension was filtered, washed with water, and then vacuum-dried at 80°C for 12 hours to obtain a metal organic framework.

### Example 1-2

Isophthalic acid in an amount of 65.0 mmol, 195 mmol of triethylamine, and 238 mL of water were mixed to prepare a solution A. Al₂(SO₄)₃·nH₂O (n = 14 to 18) in an amount of 33.8 mmol and 31.5 mL of water were mixed to prepare a solution B. The solution B was added into a reactor, and the solution A was added dropwise into the reactor at 20 to 30°C over 30 minutes. Thereafter, the mixture in the reactor was heated at 90°C for 12 hours to obtain a suspension. A portion of the obtained suspension was subjected to pressure filtration to determine the specific filtration resistance. The remaining suspension was filtered, washed with water, and then vacuum-dried at 80°C for 12 hours to obtain a metal organic framework.

### Example 1-3

Isophthalic acid in an amount of 91 mmol and 200 mmol of triethylamine were dissolved in 152 ml of water to prepare a solution A. Separately, 47.3 mmol of Al₂(SO₄)₃·nH₂O (n = 14 to 18) was dissolved in 43.8 ml of water to prepare a solution B. The solution A and the solution B were added dropwise over 90 minutes to a liquid mixture C containing 70 ml of water and 72.8 mmol of triethylamine at 20 to 30°C. Thereafter, the mixture was heated at 90°C for 12 hours to obtain a suspension. A portion of the obtained suspension was subjected to pressure filtration to determine the specific filtration resistance. The remaining suspension was filtered, washed with water, and dried at 80°C for 12 hours in a vacuum drying oven to obtain a metal organic framework.

### Comparative Example 1

Isophthalic acid in an amount of 65.0 mmol, 195 mmol of triethylamine, and 238 mL of water were mixed to prepare a solution A. Al₂(SO₄)₃·nH₂O (n = 14 to 18) in an amount of 33.8 mmol and 31.5 mL of water were mixed to prepare a solution B. The solution A was added into a reactor, and the solution B was added dropwise into the reactor at 20 to 30°C over 30 minutes. Thereafter, the mixture in the reactor was heated at 90°C for 12 hours to obtain a suspension. A portion of the obtained suspension was subjected to pressure filtration to determine the specific filtration resistance. The remaining suspension was filtered, washed with water, and then vacuum-dried at 80°C for 12 hours to obtain a metal organic framework.

### Example 2

Isophthalic acid in an amount of 65.0 mmol, 129 mmol of triethylamine, 121 mL of water, and 32 mL of N,N-dimethylformamide were mixed to prepare a solution A. Al₂(SO₄)₃·nH₂O (n = 14 to 18) in an amount of 37.05 mmol and 34.5 mL of water were mixed to prepare a solution B. At 20 to 30°C, the dropwise addition of the solution A, the solution B, and 55 mmol of triethylamine into a reactor was simultaneously started, and each addition was carried out over 30 minutes. Thereafter, the mixture in the reactor was heated at 90°C for 12 hours to obtain a suspension. A portion of the obtained suspension was subjected to pressure filtration to determine the specific filtration resistance. The remaining suspension was filtered, washed with water, and then vacuum-dried at 80°C for 12 hours to obtain a metal organic framework.

### Comparative Example 2

Isophthalic acid in an amount of 65.0 mmol, 184 mmol of triethylamine, 121 mL of water, and 32 mL of N,N-dimethylformamide were mixed to prepare a solution A. Al₂(SO₄)₃·nH₂O (n = 14 to 18) in an amount of 37.05 mmol and 34.5 mL of water were mixed to prepare a solution B. The solution A was added into a reactor, and the solution B was added dropwise into the reactor at 20 to 30°C over 30 minutes. Thereafter, the mixture in the reactor was heated at 90°C for 12 hours to obtain a suspension. A portion of the obtained suspension was subjected to pressure filtration to determine the specific filtration resistance. The remaining suspension was filtered, washed with water, and then vacuum-dried at 80°C for 12 hours to obtain a metal organic framework.

### Example 3

Isophthalic acid in an amount of 104 mmol, 26 mmol of 2,5-pyrroledicarboxylic acid, 286 mmol of triethylamine, and 150 mL of water were mixed to prepare a solution A. Al₂(SO₄)₃·nH₂O (n = 14 to 18) in an amount of 67.6 mmol and 62.5 mL of water were mixed to prepare a solution B. At 20 to 30°C, the dropwise addition of the solution A, the solution B, and 104 mmol of triethylamine into a reactor was simultaneously started, and each addition was carried out over 30 minutes. Thereafter, the mixture in the reactor was heated at 90°C for 12 hours to obtain a suspension. A portion of the obtained suspension was subjected to pressure filtration to determine the specific filtration resistance. The remaining suspension was filtered, washed with water, and then vacuum-dried at 80°C for 12 hours to obtain a metal organic framework.

### Comparative Example 3

Isophthalic acid in an amount of 104 mmol, 26 mmol of 2,5-pyrroledicarboxylic acid, 390 mmol of triethylamine, and 150 mL of water were mixed to prepare a solution A. 67.6 mmol of Al₂(SO₄)₃·nH₂O (n = 14 to 18) and 62.5 mL of water were mixed to prepare a solution B. The solution A was added into a reactor, and the solution B was added dropwise into the reactor at 20 to 30°C over 30 minutes. Thereafter, the mixture in the reactor was heated at 90°C for 12 hours to obtain a suspension. A portion of the obtained suspension was subjected to pressure filtration to determine the specific filtration resistance. The remaining suspension was filtered, washed with water, and then vacuum-dried at 80°C for 12 hours to obtain a metal organic framework.

### Example 4

Isophthalic acid in an amount of 72.8 mmol, 18.2 mmol of 2,5-pyrroledicarboxylic acid, 32 g of a 25 mass% aqueous sodium hydroxide solution, and 105 mL of water were mixed to prepare a solution A. Al₂(SO₄)₃·nH₂O (n = 14 to 18) in an amount of 47.3 mmol and 43.8 mL of water were mixed to prepare a solution B. Water in an amount of 70 g was added into a reactor, and at 20 to 30°C, the dropwise addition of the solution A, the solution B, and 11.7 g of a 25 mass% aqueous sodium hydroxide solution into the reactor was simultaneously started, and each addition was carried out over 30 minutes. Thereafter, the mixture in the reactor was heated at 90°C for 12 hours to obtain a suspension. A portion of the obtained suspension was subjected to pressure filtration to determine the specific filtration resistance. The remaining suspension was filtered, washed with water, and then vacuum-dried at 80°C for 12 hours to obtain a metal organic framework.

### Comparative Example 4

Isophthalic acid in an amount of 72.8 mmol, 18.2 mmol of 2,5-pyrroledicarboxylic acid, 43.7 g of a 25 mass% aqueous sodium hydroxide solution, and 175 mL of water were mixed to prepare a solution A. Al₂(SO₄)₃·nH₂O (n = 14 to 18) in an amount of 47.3 mmol and 43.8 mL of water were mixed to prepare a solution B. The solution A was added into a reactor, and the solution B was added dropwise into the reactor at 20 to 30°C over 30 minutes. Thereafter, the mixture in the reactor was heated at 90°C for 12 hours to obtain a suspension. A portion of the obtained suspension was subjected to pressure filtration to determine the specific filtration resistance. The remaining suspension was filtered, washed with water, and then vacuum-dried at 80°C for 12 hours to obtain a metal organic framework. The results are shown in Table 1.

### Example 5

Terephthalic acid in an amount of 84.5 mmol, 186 mmol of triethylamine, and 148 mL of water were mixed to prepare a solution A. Al₂(SO₄)₃·nH₂O (n = 14 to 18) in an amount of 43.9 mmol and 40.6 mL of water were mixed to prepare a solution B. Water in an amount of 55.7 g was added into a reactor, and at 20 to 30°C, the dropwise addition of the solution A, the solution B, and 67.6 mmol of triethylamine into the reactor was simultaneously started, and each addition was carried out over 30 minutes. Thereafter, the mixture in the reactor was heated at 90°C for 12 hours to obtain a suspension. A portion of the obtained suspension was subjected to pressure filtration to determine the specific filtration resistance. The remaining suspension was filtered, washed with water, and then vacuum-dried at 80°C for 12 hours to obtain a metal organic framework.

### Comparative Example 5

Terephthalic acid in an amount of 84.5 mmol, 253.6 mmol of triethylamine, and 203.7 mL of water were mixed to prepare a solution A. Al₂(SO₄)₃·nH₂O (n = 14 to 18) in an amount of 43.9 mmol and 40.6 mL of water were mixed to prepare a solution B. The solution A was added into a reactor, and the solution B was added dropwise into the reactor at 20 to 30°C over 30 minutes. Thereafter, the mixture in the reactor was heated at 90°C for 12 hours to obtain a suspension. A portion of the obtained suspension was subjected to pressure filtration to determine the specific filtration resistance. The remaining suspension was filtered, washed with water, and then vacuum-dried at 80°C for 12 hours to obtain a metal organic framework.

### Example 6

Isophthalic acid in an amount of 91 mmol and 200 mmol of triethylamine were dissolved in 152 ml of water to prepare a solution A. Separately, 47.3 mmol of Al₂(SO₄)₃·nH₂O (n = 14 to 18) was dissolved in 43.8 ml of water to prepare a solution B. At 20 to 30°C, the solution A and the solution B were added dropwise over 90 minutes to a liquid mixture C containing 70 ml of water and 9.2 ml of a 25wt% aqueous sodium hydroxide solution. Thereafter, the mixture was heated at 90°C for 12 hours to obtain a suspension. A portion of the obtained suspension was subjected to pressure filtration to determine the specific filtration resistance. The remaining suspension was filtered, washed with water, and dried at 80°C for 12 hours in a vacuum drying oven to obtain a metal organic framework.

The results of the specific filtration resistance and BET specific surface area of the metal organic framework obtained in each of Examples and Comparative Examples are shown in Table 1 together with the production conditions of the metal organic framework. In Table 1, the ratios shown for Examples in which two types of polyvalent carboxylic acids A were used represent molar ratios, and the ratios shown for Examples in which two types of solvents were used represent mass ratios. In addition, NEt₃ as the base C represents triethylamine, and DMF as the solvent D represents N,N-dimethylformamide. Furthermore, the numerical value shown in parentheses after the used base C represents the molar ratio of the base C to the polyvalent carboxylic acid A.

**[Table 1]**

| | Polyvalent carboxylic acid A | Metal compound B | Base C | | Solvent D | Production conditions | | pH | | | Specific filtration resistance αₘ (m/kg) | BET specific surface area (m²/g) |
|---|---|---|---|---|---|---|---|---|---|---|---|---|
| | | | Base (C1) mixed with polyvalent acid | Other base (C 2) | | Addition procedure | Temperature-holding conditions | Aqueous Solution of Polyvalent Carboxylate | Aqueous Solution of Metal Salt | Maximum Value During Contact of 0.35 Equivalent or More and Less Than 0.85 Equivalent | | |
| Example 1-1 | Isophthalic acid | Al₂(SO₄)₃ | NEt₃(2.2) | NEt₃(0.8) | Water | Acid-base mixture +Metal compound B + Base C2 added simultaneously | 90°C | 10.2 | 2.04 | 4.0 | 3.0x10¹⁰ | 605 |
| Example 1-2 | | | NEt₃(3.0) | | | Acid-base mixture →Metal compound B | | | | 4 | 9.5x10¹⁰ | 617 |
| | | | | | | | 12h | | | | | |
| Example 1-3 | | | NEt₃(2.2) | NEt₃(0.8) | | Acid-base mixture + Metal compound B →Base C2 | | 10.2 | | 5.2 | 1.0x10¹¹ | 648 |
| Comparative example 1 | | | NEt₃(3.0) | | | Metal compound B →Acid-base mixture | | 10.2 | | 8.6 | 1.3x10¹¹ | 621 |
| Example2 | Isophthalic acid | Al₂(SO₄)3 | NEt₃(2.2) | NEt₃(0.6) | Water/DMF =8/2 | Acid-base mixture +Metal compound B + Base C2 added simultaneously | 90°C | 5.8 | 2.04 | 3.7 | 9.3x10¹⁰ | 668 |
| | | | | | | | 12h | | | | | |
| Comparative example 2 | | | NEt₃(2.8) | | | Metal compound B →Acid-base mixture | | | | more than 6.5 | 6.0x10¹¹ | 658 |
| Example 3 | Isophthalic acid/ 2,5-pyrroledicarboxylic acid =8/2 | Al₂(SO₄)₃ | NEt₃(2.2) | NEt₃(0.8) | Water | Acid-base mixture +Metal compound B + Base C2 added simultaneously | 90°C | 9.8 | 2.04 | 6 or less | 6.6x10¹⁰ | 759 |
| | | | | | | | 12h | | | | | |
| Comparative example 3 | | | NEt₃(2.8) | | | Metal compound B →Acid-base mixture | | | | more than 6.5 | 8.9x10¹¹ | 746 |
| Example 4 | Isophthalic acid/ 2,5-pyrroledicarboxylic acid =8/2 | Al₂(SO₄)₃ | NaOH(2.7) | NaOH(1.0) | Water | Acid-base mixture +Metal compound B + Base C2 added simultaneously | 90°C | 12.7 | 2.04 | 4.4 | 3.6x10¹⁰ | 774 |
| | | | | | | | 12h | | | | | |
| Comparative example 4 | | | NaOH(3.7) | | | Metal compound B →Acid-base mixture | | | | 10.8 | 6.1x10¹¹ | 718 |
| Example 5 | Terephthalic acid | Al₂(SO₄)₃ | NEt₃(2.2) | NEt₃(0.6) | Water | Acid-base mixture +Metal compound B + Base C2 added simultaneously | 100°C | 10.5 | 2.04 | 4.6 | 2.7x10¹² | 143 |
| | | | | | | | 10h | | | | | |
| Comparative example 5 | | | NEt₃(2.8) | | | Metal compound B →Acid-base mixture | | | | 8.1 | 4.1x10¹² | 122 |
| Example 6 | Isophthalic acid | Al₂(SO₄)₃ | NEt₃(2.2) | NaOH(0.8) | Water | Acid-base mixture + Metal compound B →Base C2 | 90°C | 10.2 | 2.04 | 5.2 | 5.5x10¹⁰ | 639 |
| | | | | | | | 12h | | | | | |

In addition, the maximum pH values during the period from the 0.3 equivalent contact to the 0.8 equivalent contact (i.e., from the 0.25 equivalent contact to the 0.85 equivalent contact) were as follows: Example 1-1: 4.00; Example 1-2: 4.00; Comparative Example 1: 9.60; Example 2: 3.74; Comparative Example 2: more than 6; Example 3: 6 or less; Comparative Example 3: more than 6; Example 4: 4.41; Comparative Example 4: 11.50; Example 5: 4.63; and Comparative Example 5: 9.60.

The difference between the maximum pH value and the minimum pH value during the period from the 0.3 equivalent contact to the 0.8 equivalent contact (i.e., from the 0.25 equivalent contact to the 0.85 equivalent contact) was as follows: Example 1-1: 0.14; Example 1-2: 0.79; Comparative Example 1: 5.40; Example 2: 0.08; Example 4: 0.11; Comparative Example 4: 7.50; Example 5: 0.1; and Comparative Example 5: 4.70.

The difference between the maximum pH value and the minimum pH value during the period from the 0.35 equivalent contact to the 0.85 equivalent contact was as follows: Example 1-1: 0.11; Example 1-2: 0.63; Comparative Example 1: 4.4; Example 2: 0.08; Example 4: 0.11; Comparative Example 4: 6.6; Example 5: 0.07; and Comparative Example 5: 3.2.

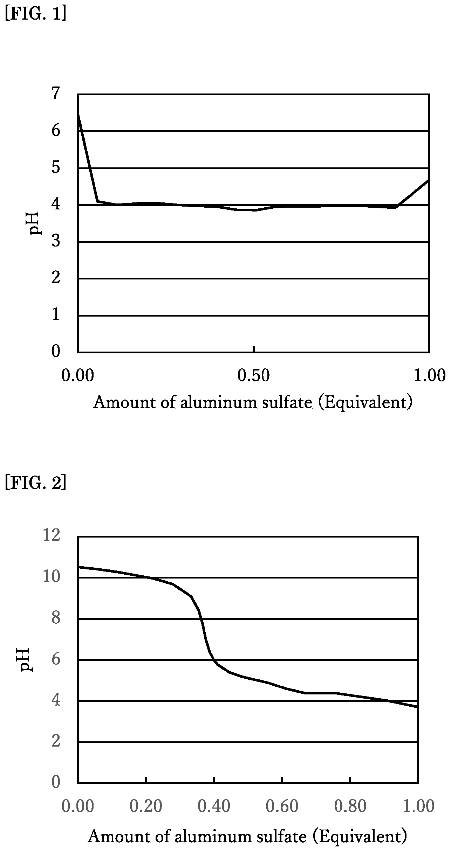

Furthermore, with respect to Example 1-1 and Comparative Example 1, graphs showing the relationship between the amount of Al₂(SO₄)₃·nH₂O (n = 14 to 18), which is the metal compound B, and the pH are shown in FIG. 1 and FIG. 2, respectively. As shown in FIG. 1, it can be seen that, in Example 1-1, the pH of the mixture in the reaction vessel had become 6 or less by the time 0.3 equivalents of the metal compound B came into contact with 0.3 equivalents of the polyvalent carboxylic acid A, and the pH of the mixture in the reaction vessel could be maintained at 6 or less until 0.8 equivalents of the metal compound B came into contact with 0.8 equivalents of the polyvalent carboxylic acid A. From Table 1, it is understood that the specific filtration resistance of the metal organic framework obtained in Example 1-1 was reduced. In addition, as shown in FIG. 2, it can be seen that, in Comparative Example 1, the pH at the time when 0.3 equivalents of the metal compound B came into contact with 0.3 equivalents of the polyvalent carboxylic acid A failed to be adjusted to 6 or less. From Table 1, it is understood that the specific filtration resistance of the metal organic framework obtained in Comparative Example 1 was high.

## Claims

1. A process for producing a metal organic framework, comprising:
continuously or intermittently adding, to a reaction vessel, one or more selected from A and B, A being a polyvalent carboxylic acid or an alkali metal salt of a polyvalent carboxylic acid, and B being a metal compound; and
mixing A, which is the polyvalent carboxylic acid or the alkali metal salt of the polyvalent carboxylic acid, and the metal compound B, in the reaction vessel in a solvent D containing water in the presence of a base C, wherein
a pH of a mixture in the reaction vessel is adjusted to 6 or less by the time 0.3 equivalents of the metal compound B come into contact with 0.3 equivalents of A, which is the polyvalent carboxylic acid or the alkali metal salt of the polyvalent carboxylic acid, in the reaction vessel, and the pH of the mixture in the reaction vessel is maintained at 6 or less until 0.8 equivalents of the metal compound B come into contact with 0.8 equivalents of A, which is the polyvalent carboxylic acid or the alkali metal salt of the polyvalent carboxylic acid.

2. The process according to claim 1, wherein
a difference between a maximum pH value and a minimum pH value during a period from when 0.3 equivalents of the metal compound B come into contact with 0.3 equivalents of A, which is the polyvalent carboxylic acid or the alkali metal salt of the polyvalent carboxylic acid, to when 0.8 equivalents of the metal compound B come into contact with 0.8 equivalents of A, which is the polyvalent carboxylic acid or the alkali metal salt of the polyvalent carboxylic acid, is 4 or less.

3. The process according to claim 1, wherein an acid-base mixture obtained by mixing A, which is the polyvalent carboxylic acid or the alkali metal salt of the polyvalent carboxylic acid, with at least a portion of the base C is added continuously or intermittently to the reaction vessel.

4. The process according to claim 1, wherein
an acid-base mixture containing A, which is the polyvalent carboxylic acid or the alkali metal salt of the polyvalent carboxylic acid, and a portion of the base C, is added continuously or intermittently to the reaction vessel via a first addition port, and a portion of the base C is added continuously or intermittently to the reaction vessel via a second addition port while addition of the acid-base mixture is continued.

5. The process according to claim 3 or 4, wherein the metal compound B is previously charged into the reaction vessel, and the acid-base mixture is added to the reaction vessel.

6. The process according to claim 3 or 4, wherein the metal compound B is added continuously or intermittently to the reaction vessel via a third addition port while the addition of the acid-base mixture is continued.

7. The process according to claim 1, wherein
an acid-base mixture containing A, which is the polyvalent carboxylic acid or the alkali metal salt of the polyvalent carboxylic acid, and at least a portion of the base C, is added continuously or intermittently to the reaction vessel via a first addition port, and the metal compound B is added continuously or intermittently to the reaction vessel via a third addition port while addition of the acid-base mixture is continued.

8. The process according to claim 1, wherein a content of water in the solvent D is 70 mass% or more, based on 100 mass% of the solvent D.
